# FASCICULE DE BREVET EUROPEEN

(11) **EP 3 481 286 B1**
(45) Date de publication et mention de la délivrance du brevet: **13.12.2023**
(21) Numéro de dépôt: 17746194.4
(22) Date de dépôt: 06.07.2017
(51) Int. Cl.: A61B 5/00, G01C 22/00, H04B 10/116, G16H 20/30, G16H 30/40, G16H 40/63, G16H 40/67

(54) **PROCÉDÉ ET SYSTÈME DE MESURE ET D'AFFICHAGE DE DONNÉES LIÉES À L'ACTIVITÉ PHYSIQUE D'UNE PERSONNE**
VERFAHREN UND SYSTEM ZUR MESSUNG UND ANZEIGE VON DATEN IN ZUSAMMENHANG MIT DER PHYSISCHEN AKTIVITÄT EINER PERSON
METHOD AND SYSTEM FOR MEASURING AND DISPLAYING DATA LINKED TO A PERSON'S PHYSICAL ACTIVITY

(30) Priorité: 07.07.2016 CH 8722016; 24.02.2017 CH 2162017
(43) Date de publication de la demande: 15.05.2019
(73) Titulaire: Guenat SA Montres Valgine, 2345 Les Breuleux (CH)
(72) Inventeur: GARINAUD, Frédéric, 2340 Le Noirmont (CH)
(74) Mandataire: P&TS SA (AG, Ltd.)
(86) Numéro de dépôt international: PCT/IB2017/054084
(87) Numéro de publication internationale: WO 2018/007978

(56) Documents cités:
- EP-A1- 1 553 469
- EP-A1- 2 458 458
- US-A1- 2013 234 850

## Description

### Domaine technique

La présente invention concerne la mesure de l'activité physique de personnes à l'aide de montres-bracelets.

### Etat de la technique

On connaît dans l'état de la technique des montres-bracelets et d'autres dispositifs portés au poignet qui permettent de déterminer l'activité physique du porteur. Certaines montres électroniques sont par exemple munies d'un compteur de pas qui permet de déterminer le nombre de pas effectués par le porteur. D'autres montres de sport permettent d'afficher la distance parcourue pendant une course, le dénivelé, ou d'autres paramètres liés à l'activité physique.

Ces paramètres peuvent être affichés sur l'écran de la montre ou du bracelet. Les montres connectées (« smartwatches ») permettent en outre de les transmettre, par exemple via une interface filaire ou sans fil, vers un ordinateur ou un smartphone afin de les afficher et de les manipuler plus confortablement. On connaît ainsi des montres connectées qui communiquent via une interface Bluetooth avec un smartphone à proximité. Ce smartphone permet d'afficher les résultats et de les mémoriser.

WO13134713 préconise une communication entre une montre électronique munie de capteurs biométriques et un smartphone est effectuée grâce à un protocole de communication optique. Le capteur d'image du smartphone capture une image du cadran de la montre présentant un tag, par exemple sous la forme d'un QR code, et en extrait des données.

Ces solutions sont cependant réservées à des montres électroniques et qui disposent d'une batterie pour alimenter l'interface de communication. Elles ne sont donc pas applicables à des montres mécaniques comportant uniquement des indicateurs analogiques, par exemple des aiguilles ou des disques tournants.

De nombreux consommateurs apprécient cependant l'élégance des montres mécaniques et le fait qu'elles ne doivent pas être rechargées ni même remontées dans le cas de montres automatiques. Les montres mécaniques ne se contentent d'ailleurs pas d'afficher l'heure courante ; elles comportent souvent d'autres complications pour mesurer et afficher des paramètres non liés à l'heure courante. A l'heure actuelle, ces paramètres complémentaires sont uniquement affichés et traités dans la montre, sans le confort et la puissance qu'offre un traitement électronique dans un ordinateur ou un smartphone.

### Bref résumé de l'invention

Un but de la présente invention est donc de proposer un procédé permettant d'afficher sur un équipement électronique des données non liées au temps et mesurées avec une montre mécanique.

En particulier, un but de la présente invention est donc de proposer un procédé permettant d'afficher sur un équipement électronique des données liées à l'activité physique et mesurées avec une montre mécanique.

Selon un aspect, ce but est obtenu grâce à un procédé de mesure et d'affichage de données représentatives de l'activité physique d'une personne, comprenant :
- -mesure de données représentative de l'activité physique de la personne au moyen d'un capteur mécanique dans une montre bracelet portée par la personne ;
- affichage de ces données mesurées sur le cadran de la montre-bracelet au moyen d'un indicateur analogique ;
- capture d'image dudit cadran au moyen d'un appareil photo dans un équipement électronique portable;
- analyse de ladite image pour déterminer la position dudit indicateur analogique, ladite position constituant une ou des données analysées;
- traitement dans l'équipement électronique portable de ladite ou desdites données analysées, de manière à déterminer des données représentatives de l'activité physique traitées ;
- affichage des données représentatives de l'activité physique traitées sur un écran de l'équipement électronique.

L'indicateur analogique peut comporter par exemple une aiguille ou un disque tournant.

Le procédé met donc en oeuvre un appareil photo et un équipement électronique afin de capturer une image de la montre mécanique et de déterminer la position de l'indicateur analogique, et donc les données mesurées, grâce à une analyse d'images.

L'équipement électronique portable peut être par exemple un smartphone, une tablette, une montre intelligente électronique (smartwatch), une lunette connectée, ou un autre équipement personnel destiné à être emporté par un utilisateur lors de ses activités quotidiennes, par exemple en le portant sur le corps ou dans une poche.

Cette solution présente notamment l'avantage par rapport à l'art antérieur d'éviter la nécessité de composants électroniques, d'un connecteur ou d'une antenne, et d'une batterie dans la montre mécanique.

A cet effet, les données représentatives de l'activité physique peuvent par exemple être déterminées par une intégration des oscillations subies par une masse oscillante depuis un instant de mise à zéro, ou par unité de temps.

Les données affichées sur le cadran de la montre sont donc déterminées de manière purement mécanique. En raison des limitations de la mécanique, la précision de ces données est limitée, et la nature des données est restreinte.

La limitation de la précision provient par exemple de la variabilité due au processus de fabrication, des facteurs environnementaux (température, gravité, chocs), etc

Les restrictions liées à la nature des données proviennent à la fois de la difficulté qu'il y a à réaliser des calculs complexes avec des moyens mécaniques. L'affichage mécanique dans la montre est néanmoins très utile comme première indication, toujours disponible même en l'absence toute source d'alimentation électrique ou lorsque l'équipement électronique portable n'est pas disponible.

Le traitement des données par l'électronique portable peut être utilisé pour améliorer leur précision, par exemple afin de compenser ou de corriger des erreurs prévisibles, par exemple des biais.

Le traitement des données par l'électronique portable peut être utilisé pour afficher des données représentatives de l'activité physique différentes, qui ne sont pas les données affichées sur la montre, mais qui sont déduites des données affichées par la montre.

Comme exemple d'application, un coureur peut utiliser la montre mécanique pour obtenir pendant la course une indication du nombre de pas effectués, obtenue grâce à des moyens mécaniques. Après sa course, il peut lire les données affichées sur le cadran de sa montre avec son smartphone, et obtenir d'une part une évaluation du nombre de pas plus précise (par exemple en corrigeant des biais connus ou propres à la montre), et d'autre part des données traitées additionnelles, par exemple une distance parcourue, un nombre de calories dépensé, etc.

La position de l'indicateur peut être représentée par exemple par un ou plusieurs chiffres, par exemple une valeur numérique pointée par une aiguille, par un angle par rapport à une position de référence, par l'angle de deux aiguilles, etc. On parle donc de données.

Une analyse d'image a déjà été mise en oeuvre afin de vérifier la position des aiguilles d'une montre et sa bonne marche. A titre d'exemple, WO12069444A1 concerne une méthode de détermination de l'écart de marche d'une montre mécanique avec un analyseur d'images. De la même façon, WO05003867 concerne un système de correction de l'heure d'une montre à aiguilles grâce à une caméra qui capture des images du cadran, et un système qui analyse ces images pour déterminer l'heure affichée par la montre. Ces solutions permettent uniquement de vérifier si l'heure indiquée par une montre mécanique est précise ; elles ne sont d'aucune utilité lorsqu'il s'agit de transmettre d'autres données.

Le besoin d'analyser la position des indicateurs analogiques d'une montre s'est donc uniquement fait sentir pour des besoins de corrections ou de vérification de la marche d'une montre ; il s'agit dans ce cas de comparer la mesure du temps effectuée par la montre avec une mesure supposée plus précise effectuée par un système électronique complexe. En revanche, ces documents ne décrivent pas en quoi il pourrait être utile de transmettre à un équipement électronique externe à la montre des données relatives à l'activité physique qui n'ont pas besoin ou qui ne peuvent pas être vérifiées.

En tous les cas, cet art antérieur ne suggère pas de montre bracelet comportant un capteur mécanique de l'activité physique. Les compteurs d'activité physique usuels, notamment les compte-pas, emploient conventionnellement un accéléromètre électronique, par exemple basé sur un circuit MEMS.

Par données représentatives de l'activité physique, on entend dans la présente demande des données dont la fonction première est de renseigner le porteur de la montre sur les efforts consentis lors d'une activité physique. Des données sont dites représentatives de l'activité physique d'une personne lorsqu'elles sont destinées à déterminer le niveau d'activité physique de la personne et de lui indiquer si cette activité est suffisante, par exemple dans le cadre d'un entraînement ou d'une mise en forme, ou si elle doit être augmentée. Les données physiques peuvent comporter par exemple une estimation du nombre de pas effectués, d'une distance parcourue, d'une énergie fournie depuis le début de la mesure, ou d'une énergie par unité de temps.

Par capteur mécanique, on entend dans la présente invention un capteur dont le fonctionnement est exclusivement mécanique et qui ne requiert donc pas de composant électrique ou électronique.

Le procédé comporte avantageusement les étapes suivantes :
stockage de paramètres propres à la personne dans un espace mémoire ;
ledit traitement de données utilisant lesdits paramètres propres à la personne pour déterminer lesdites données représentatives de l'activité physique traitées de manière personnalisée pour la personne.

L'espace mémoire peut être un espace mémoire de l'équipement électronique portable, ou un espace mémoire accessible par l'équipement ou le module de traitement des données.

Ce procédé permet ainsi une mesure de paramètres physiques entièrement mécanique, au moyen d'un capteur mécanique dans une montre bracelet mécanique, puis une détermination des données affichées personnalisée en fonction de l'utilisateur grâce à la souplesse de traitement de l'équipement électronique portable.

Dans un mode de réalisation, les données mesurées dans la montre bracelet mécanique correspondent à des oscillations ou à des alternances d'une masse oscillante. La montre bracelet affiche une grandeur qui dépend de ce nombre d'oscillations ou d'alternances, et qui peut par exemple correspondre à un nombre de pas. Le dispositif électronique portable détermine la valeur indiquée par analyse d'image, puis la traite en fonction de paramètres personnalisés tels que par exemple la taille de l'utilisateur, la longueur moyenne de ses enjambées, son poids, etc, afin de déterminer et d'afficher d'autres données traitées, par exemple la distance parcourue, le nombre de calories dépensées, le nombre d'étages grimpés, etc.

Le procédé comporte avantageusement les étapes suivantes :
stockage de paramètres de calibration propres à la montre bracelet dans un espace mémoire ;
ledit traitement de données utilisant lesdits paramètres de calibration propres à la montre bracelet pour déterminer lesdites données représentatives de l'activité physique calibrées pour la montre bracelet.

L'équipement électronique portable peut ainsi corriger d'éventuelles erreurs de mesure propres à la montre bracelet.

Les paramètres de calibration peuvent par exemple être déterminés en comparant une série de mesures effectuées avec la montre bracelet et des mesure comparables effectuées avec le dispositif électronique portable.

Le capteur mécanique peut être un capteur de mouvement mécanique intégré à la montre bracelet.

Le capteur mécanique peut être un capteur qui compte le nombre d'oscillations.

Le capteur mécanique peut être un capteur qui tient compte du nombre et de l'amplitude des oscillations.

Le capteur mécanique peut être un capteur d'oscillations lors de la marche.

Le capteur de mouvement peut être un podomètre. Les données dépendent alors du nombre de pas effectués par la personne.

Le procédé peut comprendre une étape de mise à zéro automatique et périodique des données affichées sur le cadran.

Le procédé peut comprendre une étape de mise à zéro manuelle des données affichées sur le cadran.

Dans un mode de réalisation, les données affichées sont incrémentées à chaque mouvement du porteur ou à chaque mouvement du porteur dans une direction ou un sens donné, et uniquement décrémentées par une action volontaire de l'utilisateur ou de manière automatique à intervalles périodiques.

L'invention a aussi pour objet un système de mesure et d'affichage de données représentatives de l'activité physique d'une personne, comprenant :
- une montre-bracelet munie d'un capteur mécanique pour capturer des données liées à l'activité physique de la personne lorsqu'elle porte la montre-bracelet, et d'un indicateur analogique pour afficher ces données sur un cadran de la montre-bracelet;

un équipement électronique portable muni d'un appareil photo;
un module d'analyse d'images capturées par ledit appareil photo, permettant de déterminer les données affichées sur le cadran lorsque l'image comprend une photo du cadran ;
un module de traitement pour traiter lesdites données déterminées par le module d'analyse d'image ;
un module d'affichage des données traitées sur un écran.

Selon un aspect, l'invention a aussi pour objet un support de données informatique comportant un programme exécutable par un équipement électronique afin que ledit équipement traite et affiche des données liées à l'activité physique d'une personne, ledit programme comprenant :
un module de capture d'images ;
un module d'analyse d'images capturées, permettant de déterminer des données affichées sur un cadran lorsque l'image comprend une photo du cadran ;
un module de traitement pour traiter lesdites données déterminées par le module d'analyse d'image ;
un module d'affichage des données traitées sur un écran.

L'équipement électronique peut être un smartphone, une tablette, un ordinateur, etc.

Les modes de réalisations décrits pour le procédé selon l'invention s'appliquent au système et au support de données selon l'invention et vice versa, mutatis mutandis.

### Brève description des figures

Des exemples de mise en oeuvre de l'invention sont indiqués dans la description illustrée par les figures annexées dans lesquelles :
- La figure 1 illustre un exemple de système selon l'invention.
- La figure 2 est un schéma-bloc des principaux modules d'un appareil selon l'invention.

### Exemple(s) de mode de réalisation de l'invention

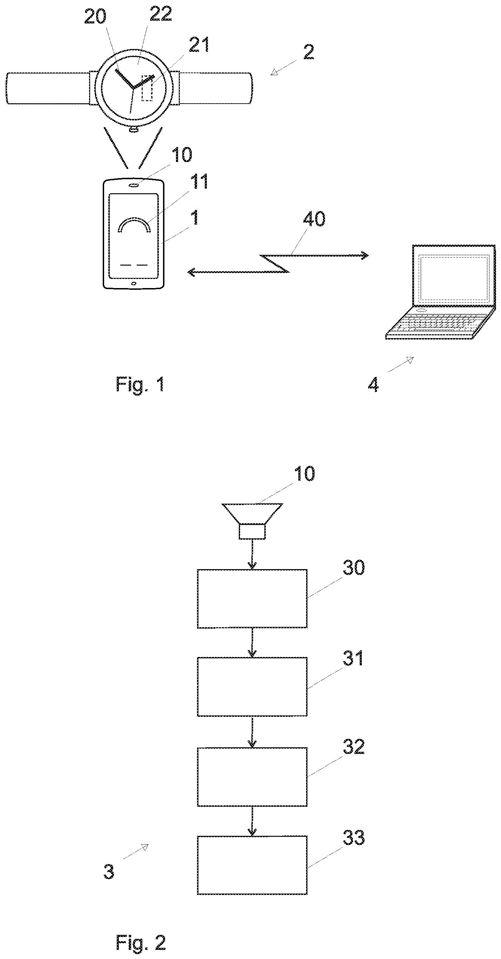

Un exemple de système selon la présente invention est illustré sur la figure 1. Le système comporte un équipement électronique 1 et une montre-bracelet mécanique 2.

L'équipement électronique 1 peut être par exemple un smartphone, une tablette etc., muni d'un écran 11 et d'un appareil photo 10 pour capturer des images fixes ou animées (vidéos). Une interface filaire ou sans fil optionnelle, par exemple une interface wifi ou Bluetooth, permet de le connecter à un équipement externe, par exemple un ordinateur, et/ou à Internet.

La montre-bracelet 2 est munie d'un indicateur analogique 20, par exemple d'un indicateur à aiguilles, à disques, à rouleaux, à balises escamotables, ou avec une autre pièce du mouvement, pour afficher l'heure courante ainsi que des données non liées au temps, par exemple des données liées à l'activité physique de la personne qui porte la montre.

Ces données sont obtenues au moyen d'un capteur mécanique 21 intégré à la montre bracelet. Le capteur peut faire partie du mouvement de la montre, ou d'un module auxiliaire superposé sur le mouvement principal.

Dans un mode de réalisation, ces données dépendent d'accélérations de la montre lorsque le porteur de la montre bouge son poignet. Il est possible de mesurer par exemple l'accélération maximale depuis un instant de remise à zéro, qui donne une indication de force, ou l'accélération cumulée qui donne une indication du travail effectué.

Ces accélérations peuvent être mesurées avec une masse de test dans le capteur mécanique, c'est-à-dire une masse mise en rotation, en oscillation ou en translation par les mouvements de la personne. Cette masse peut être une masse différente de la masse oscillante chargée du remontage automatique de la montre. Dans un mode de réalisation, cette masse est la masse oscillante du mouvement, dont les oscillations sont comptées mécaniquement afin d'en tirer des informations, autres que la réserve de marche, et relatives à l'activité physique du porteur.

Un intégrateur non représenté additionne les oscillations ou les alternances subies par la masse de test pour en déduire des données représentatives de l'activité de la personne. Dans un mode de réalisation, cet intégrateur comporte un ressort, par exemple un ressort spiral, ou un autre élément déformable, qui se tend à chaque oscillation de la masse de test, ou à chaque oscillation dont l'amplitude dépasse un seuil, ou à chaque oscillation dans un sens donné. L'élément déformable se détend automatiquement, par exemple chaque jour à minuit, ou manuellement sous l'action d'un bouton-poussoir, ou en continu. La tension du ressort dépend alors du nombre d'oscillations depuis la mise à zéro manuelle ou automatique, et éventuellement de l'amplitude de ces oscillations; elle peut être affichée pour indiquer une mesure de l'énergie dépensée par l'utilisateur depuis la mise à zéro.

Il est aussi possible d'utiliser un compteur en tant qu'intégrateur, par exemple un compteur numérique, dont la valeur est incrémentée à chaque oscillation.

Dans ces modes de réalisation, les données représentent donc une intégration des accélérations subies par la montre-bracelet 2 pendant une période donnée. Ces données peuvent par exemple dépendre du nombre de pas effectués par la personne.

Un module de remise à zéro non représenté permet de remettre le compteur à zéro périodiquement, par exemple à chaque fois que l'heure indiquée par le mouvement de la montre correspond à minuit, ou à chaque fois que le mouvement passe d'une phase d'activité intense à une phase d'activité moins intense, ou vice-versa. Un module de remise à zéro manuelle, par exemple à l'aide d'un bouton-poussoir ou d'une position de la couronne, peut aussi être utilisé. Il est aussi possible de remettre à zéro un compteur lorsque l'utilisateur retire sa montre, par exemple pour dormir, et qu'aucun mouvement n'est détecté pendant une durée déterminée.

Les données mesurées affichées sont affichées sur le cadran 22, par exemple au moyen d'une aiguille dédiée à cet affichage, ou d'un autre indicateur analogique approprié. Il est aussi possible d'afficher une valeur au moyen de plusieurs aiguilles.

L'équipement électronique portable comporte un programme électronique, par exemple une app ou un autre exécutable, décrite en relation avec la figure 2. L'élément 10 correspond à l'appareil photo déjà décrit. Un module de capture d'image 30, qui peut être distinct du programme, permet de capturer des images du cadran 22, ou d'autres images.

Un module d'analyse d'image 31 reçoit les images capturées avec l'appareil photo 10 et les analyse au moyen d'un algorithme de reconnaissance d'image, afin de déterminer les données liées à l'activité de la personne affichées de manière analogique sur le cadran lorsque l'image analysée comprend une photo de ce cadran.

A cet effet, le module 31 peut comprendre un module de prétraitement pour ajuster le contraste, la luminosité, et/ou la balance des blancs de l'image. Le prétraitement peut aussi inclure une correction de perspective au cas où l'image est prise depuis une direction non perpendiculaire au cadran, ce qui permet de corriger les erreurs de parallaxe ; à cet effet, il peut être avantageux de prévoir une forme de référence sur la cadran 22, par exemple une forme rectangulaire ou autre, connue par le module de prétraitement qui peut corriger les déformations en trapèze pour redresser les erreurs de perspective.

Un module de recadrage isole et oriente la portion d'image redressée comportant le cadran 22, ou en tout cas l'indicateur (par exemple l'aiguille et les index associés) qui affiche les données pertinentes sur ce cadran.

Le module d'analyse 31 comprend aussi un module d'extractions d'éléments (« feature extraction » en anglais) afin d'extraire les éléments recherchés de l'image, notamment les indicateurs, par exemple les aiguilles ou l'extrémité de celles-ci. Un module de détermination de la position de ces features permet ensuite de déterminer l'angle de l'aiguille identifiée, ou la position angulaire ou longitudinale d'un autre indicateur, pour en déduire directement les données d'activité physique affichées.

Les données ainsi déterminées sont transmises à un module de traitement 32 qui les traite afin de déterminer des données traitées, puis de les afficher.

Le traitement peut par exemple comprendre une correction des données affichées sur la montre bracelet. La correction peut tenir compte par exemple de données de calibration préalablement déterminées et propres à la montre-bracelet et/ou à la personne. Dans un mode de réalisation, le procédé comporte une étape préalable de comparaison des paramètres physiques mesurés par la montre bracelet et par l'équipement électronique portable lors d'un même effort physique, puis la détermination d'une table ou d'une fonction de correction selon les résultats de cette comparaison. Il est ainsi possible de corriger un biais systématique dans la mesure de l'équipement électronique portable.

Le traitement peut inclure la détermination de valeurs personnalisées en fonction de la personne, à l'aide de paramètres personnalisés stockés dans un espace mémoire de la montre bracelet. Dans un mode de réalisation, le module de traitement utilise à cet effet des données introduites préalablement par le porteur de la montre, par exemple son poids, sa taille, la longueur de ses enjambées, son âge, son genre, ses objectifs, le type d'activité physique, etc. A partir d'un nombre de pas affichés sur la montre bracelet et de ces paramètres personnalisés, il est ainsi possible de déterminer par exemple une distance parcourue ou un nombre de calories consommées, selon un calcul personnalisé pour la personne.

Le traitement peut aussi inclure par exemple le stockage, la comparaison avec des données antérieures ou avec des données de référence, et/ou le calcul d'autres données à partir des données brutes ainsi déterminées.

D'autres données employées par le module 32, telles que des données météorologiques, des comparaisons avec d'autres utilisateurs, etc., peuvent être tirées d'un réseau de communication, par exemple Internet.

Un module d'affichage 33 permet ensuite d'afficher les données ainsi traitées sur un écran 11 de l'équipement électronique 1, ou sur un autre écran. Les données peuvent en outre être restituées sous une forme sonore.

Les données mesurées et affichées peuvent par exemple comprendre un nombre de pas, un index d'activité (ou d'inactivité) ou un nombre de calories effectués respectivement dépensées depuis la dernière mise à zéro du compteur de la montre. Dans une variante, il est possible d'afficher un pourcentage d'activité par rapport à un objectif défini par l'utilisateur ou proposé par le programme.

Plusieurs affichages peuvent être prévus. Par exemple, un premier affichage peut indiquer une donnée représentative de nombre et de l'amplitude des oscillations depuis un instant de mise à zéro ; la valeur dépend donc de l'énergie physique dépensée, ou du nombre de pas effectués, depuis cette mise à zéro, par exemple depuis minuit. Un deuxième affichage peut par exemple être incrémenté à chaque déplacement, et décrémenté régulièrement avec l'écoulement du temps mesuré par le mouvement de la montre ; la valeur affichée correspond donc à une puissance dépensée.

Il est aussi possible de calculer et d'afficher un nombre d'heures de sommeil, d'activité physique légère, ou d'activité physique intense. Une phase de sommeil peut être détectée par le capteur mécanique et/ou par le module de traitement lorsque le capteur de mouvement détecte une faible activité physique pendant une période prolongée ; la probabilité qu'il s'agisse d'heures de sommeil est encore plus grande si cette faible activité se produit la nuit. A cet effet, le capteur mécanique peut être agencé pour détecter les passages entre phases de sommeil, d'activité légère et d'activité intense ; les instants de passage, ou les durées, peuvent être affichées sur le cadran de la montre.

Ces données peuvent être affichées sous forme de texte, de chiffres et/ou de diagrammes. Elles peuvent aussi être sauvegardées ou transmises à d'autres dispositifs à distance.

## Revendications

1. Procédé de mesure et d'affichage de données liées à l'activité physique d'une personne, comprenant :
- mesure de données représentative de l'activité physique de la personne au moyen d'un capteur mécanique (21) dans une montre bracelet mécanique (2) portée par la personne, le fonctionnement dudit capteur mécanique (21) étant exclusivement mécanique ;
- affichage de ces données mesurées sur le cadran (22) de la montre-bracelet (2) au moyen d'un indicateur analogique (20) ;
- capture d'image dudit cadran au moyen d'un appareil photo (10) dans un équipement électronique portable (1);
- analyse de ladite image pour déterminer la position dudit indicateur analogique (20), ladite position constituant un ou des données analysées ;
- traitement dans l'équipement électronique portable de ladite ou desdites données analysées , de manière à déterminer des données représentatives de l'activité physique traitées ;
- affichage des données représentatives de l'activité physique traitées sur un écran (11) de l'équipement électronique (1).

2. Procédé selon la revendication 1, comportant en outre :
une étape de stockage de paramètres propres à la personne dans un espace mémoire de l'équipement électronique portable (1), ou accessible par l'équipement électronique portable (1) ou par un module de traitement des données ;
ledit traitement de données utilisant lesdits paramètres propres à la personne pour déterminer lesdites données représentatives de l'activité physique traitées de manière personnalisée pour la personne.

3. Procédé selon l'une des revendications 1 ou 2, comportant en outre :
stockage de paramètres de calibration propres à la montre bracelet mécanique dans un espace mémoire de l'équipement électronique portable (1), ou accessible par l'équipement électronique portable (1) ou par un module de traitement des données ;
ledit traitement de données utilisant lesdits paramètres de calibration propres à la montre bracelet mécanique pour déterminer lesdites données représentatives de l'activité physique calibrées pour la montre bracelet mécanique.

4. Procédé selon l'une des revendications 1 à 3, ledit capteur mécanique (21) étant un capteur de mouvement, par exemple un podomètre, ou un accéléromètre.

5. Procédé selon la revendication 4, comportant une étape d'intégration des mouvements subis par le capteur de mouvement depuis un instant de remise à zéro, et d'affichage au moyen dudit indicateur analogique d'une valeur correspondant à cette intégration.

6. Procédé selon l'une des revendications 2 à 5, lesdites données traitées en utilisant les paramètres propres à la personne indiquant une distance parcourue et/ou un nombre de calories dépensées.

7. Procédé selon l'une des revendications 1 à 6, comprenant une étape de mise à zéro automatique et périodique des données affichées sur le cadran, ou une étape de mise à zéro manuelle des données affichées sur le cadran (22).

8. Procédé selon l'une des revendications 1 à 7, dans lequel les données affichées sont incrémentées à chaque mouvement du porteur ou à chaque mouvement du porteur dans une direction ou un sens donné, et uniquement décrémentées par une action volontaire de l'utilisateur ou de manière automatique à intervalles périodiques.

9. Système de mesure et d'affichage de données liées à l'activité physique d'une personne, comprenant :
- une montre-bracelet (2) munie d'un capteur mécanique (21) pour capturer des données représentatives de l'activité physique de la personne lorsqu'elle porte la montre-bracelet, le fonctionnement dudit capteur mécanique (21) dont le fonctionnement étant exclusivement mécanique, et d'un indicateur analogique (20) pour afficher ces données sur un cadran de la montre-bracelet;
un équipement électronique portable muni d'un appareil photo (10) ;
un module d'analyse d'images (31) capturées par ledit appareil photo (10), permettant de déterminer une ou des données analysées correspondant aux données affichées sur le cadran lorsque l'image comprend une photo du cadran ;
un module de traitement (32) pour traiter ladite ou lesdites données analysées déterminées par le module d'analyse d'image et générer des données traitées ;
un module d'affichage (33) des données traitées sur un écran.

10. Système selon la revendication 9, ledit capteur mécanique (21) étant un capteur de mouvement, par exemple un podomètre ou un accéléromètre.

11. Système selon l'une des revendications 9 à 10, comprenant un organe de mise à zéro automatique et périodique des données affichées sur le cadran (22).

12. Système selon l'une des revendications 9 à 11, comprenant un organe de mise à zéro manuelle des données affichées sur le cadran (22).

13. Système selon l'une des revendications 9 à 12, comprenant :
un espace mémoire de stockage de paramètres propres à la personne dans l'équipement électronique portable (1), ou accessible par l'équipement électronique portable (1) ou par ledit module de traitement de données ;
le module de traitement de données étant agencé pour utiliser lesdits paramètres propres à la personne pour déterminer lesdites données représentatives de l'activité physique traitées de manière personnalisée pour la personne.

14. Système selon l'une des revendications 9 à 13, comportant en outre :
un espace mémoire de stockage de paramètres de calibration propres à la montre bracelet mécanique dans l'équipement électronique portable (1), ou accessible par l'équipement électronique portable (1) ou par ledit module de traitement de données ;
ledit module de traitement de données étant agencé pour utiliser lesdits paramètres de calibration propres à la montre bracelet mécanique pour déterminer lesdites données représentatives de l'activité physique calibrées pour la montre bracelet mécanique.

## Patentansprüche

1. Verfahren zum Messen und Anzeigen von Daten, die sich auf die körperliche Aktivität einer Person beziehen, umfassend:
- Messen von Daten, die für die körperliche Aktivität der Person charakteristisch sind, mittels eines mechanischen Sensors (21) in einer mechanischen Armbanduhr (2), die von der Person getragen wird, wobei die Funktion des mechanischen Sensors (21) ausschliesslich mechanischer Natur ist;
- Anzeigen dieser gemessenen Daten auf dem Zifferblatt (22) der Armbanduhr (2) mithilfe eines analogen Indikators (20);
- Erfassen eines Bildes des Zifferblatts mithilfe einer Kamera (10) in einer tragbaren elektronischen Ausrüstung (1);
- Analysieren des Bildes, um die Position des analogen Indikators (20) zu bestimmen, wobei die Position einen oder mehrere analysierte Daten darstellt;
- Verarbeiten der analysierten Daten in der tragbaren elektronischen Ausrüstung, um repräsentative Daten für die verarbeitete körperliche Aktivität zu bestimmen;
- Anzeigen der verarbeiteten repräsentativen Daten der körperlichen Aktivität auf einem Bildschirm (11) der elektronischen Ausrüstung (1).

2. Verfahren nach Anspruch 1, ferner umfassend:
einen Schritt des Speicherns von personenspezifischen Parametern in einem Speicherbereich der tragbaren elektronischen Ausrüstung (1), oder zugänglich durch die tragbare elektronische Ausrüstung (1) oder durch ein Datenverarbeitungsmodul;
wobei die Datenverarbeitung die personenspezifischen Parameter verwendet, um die repräsentativen Daten der körperlichen Aktivität zu bestimmen, die auf personenspezifische Weise verarbeitet werden.

3. Verfahren nach einem der Ansprüche 1 oder 2, ferner umfassend:
Speichern von der mechanischen Armbanduhr eigenen Kalibrierungsparametern in einem Speicherplatz der tragbaren elektronischen Ausrüstung (1), oder zugänglich durch die tragbare elektronische Ausrüstung (1) oder durch ein Datenverarbeitungsmodul;
wobei die Datenverarbeitung die für die mechanische Armbanduhr spezifischen Kalibrierungsparameter verwendet, um die für die mechanische Armbanduhr kalibrierten repräsentativen Daten der körperlichen Aktivität zu bestimmen.

4. Verfahren nach einem der Ansprüche 1 bis 3, wobei der mechanische Sensor (21) ein Bewegungssensor, beispielsweise ein Schrittzähler, oder ein Beschleunigungsmesser ist.

5. Verfahren nach Anspruch 4, umfassend einen Schritt des Integrierens der Bewegungen, denen der Bewegungssensor seit einem Zeitpunkt des Zurücksetzens auf Null ausgesetzt war, und des Anzeigens eines dieser Integration entsprechenden Werts mittels des genannten Analogindikators.

6. Verfahren nach einem der Ansprüche 2 bis 5, wobei die unter Verwendung der personenspezifischen Parameter verarbeiteten Daten eine zurückgelegte Strecke und/oder eine Anzahl verbrauchter Kalorien angeben.

7. Verfahren nach einem der Ansprüche 1 bis 6, umfassend einen Schritt des automatischen und periodischen Nullsetzens der auf dem Zifferblatt angezeigten Daten oder einen Schritt des manuellen Nullsetzens der auf dem Zifferblatt (22) angezeigten Daten.

8. Verfahren nach einem der Ansprüche 1 bis 7, wobei die angezeigten Daten bei jeder Bewegung des Trägers oder bei jeder Bewegung des Trägers in eine bestimmte Richtung oder Richtung inkrementiert werden und ausschliesslich durch eine willentliche Handlung des Nutzers oder automatisch in periodischen Intervallen dekrementiert werden.

9. System zum Messen und Anzeigen von Daten, die sich auf die körperliche Aktivität einer Person beziehen, umfassend:
- eine Armbanduhr (2) mit einem mechanischen Sensor (21) zum Erfassen von Daten, die für die körperliche Aktivität der Person beim Tragen der Armbanduhr repräsentativ sind, wobei der mechanische Sensor (21) ausschliesslich mechanisch arbeitet, und mit einem analogen Indikator (20) zum Anzeigen dieser Daten auf einem Zifferblatt der Armbanduhr;
ein tragbares elektronisches Equipment, das mit einer Kamera (10) ausgestattet ist;
ein Modul zur Analyse von Bildern (31), die von der Kamera (10) aufgenommen werden, um einen oder mehrere analysierte Daten zu bestimmen, die den auf dem Zifferblatt angezeigten Daten entsprechen, sofern das Bild ein Foto des Zifferblatts enthält;
ein Verarbeitungsmodul (32) zum Verarbeiten der analysierten Daten, die von dem Bildanalysemodul bestimmt wurden, und zum Erzeugen von verarbeiteten Daten;
ein Anzeigemodul (33) zum Anzeigen der verarbeiteten Daten auf einem Bildschirm.

10. System nach Anspruch 9, wobei der mechanische Sensor (21) ein Bewegungssensor ist, beispielsweise ein Schrittzähler oder ein Beschleunigungsmesser.

11. System nach einem der Ansprüche 9 bis 10, mit einer Instanz zur automatischen und periodischen Nullstellung der auf dem Zifferblatt (22) angezeigten Daten.

12. System nach einem der Ansprüche 9 bis 11, mit einer Instanz zur manuellen Nullstellung der auf dem Zifferblatt (22) angezeigten Daten.

13. System nach einem der Ansprüche 9 bis 12, umfassend:
einen Speicherbereich zum Speichern von personenspezifischen Parametern in der tragbaren elektronischen Ausrüstung (1) oder zugänglich durch die tragbare elektronische Ausrüstung (1) oder durch das Datenverarbeitungsmodul;
wobei das Datenverarbeitungsmodul dazu ausgebildet ist, die personenbezogenen Parameter zu verwenden, um die für die Person individuell verarbeiteten repräsentativen Daten zur körperlichen Aktivität zu bestimmen.

14. System nach einem der Ansprüche 9 bis 13, das ausserdem umfasst:
einen Speicherbereich zum Speichern von Kalibrierungsparametern, die spezifisch für die mechanische Armbanduhr sind, in der tragbaren elektronischen Ausrüstung (1) oder zugänglich durch die tragbare elektronische Ausrüstung (1) oder durch das Datenverarbeitungsmodul;
wobei das Datenverarbeitungsmodul dazu ausgebildet ist, die für die mechanische Armbanduhr spezifischen Kalibrierungsparameter zu verwenden, um die für die mechanische Armbanduhr kalibrierten repräsentativen Daten der körperlichen Aktivität zu bestimmen.

## Claims

1. Method for measuring and displaying data related to the physical activity of a person, comprising:
- measuring data representative of the person's physical activity by means of a mechanical sensor (21) in a mechanical wristwatch (2) worn by the person, the functioning of said mechanical sensor (21) being exclusively mechanical;
- displaying said measured data on the dial (22) of the wristwatch (2) by means of an analogue indicator (20);
- capturing an image of said dial by means of a camera (10) in portable electronic equipment (1);
- analysing said image to determine the position of said analogue indicator (20), said position constituting one or more analysed data;
- processing in the portable electronic equipment of said analysed data, so as to determine data representative of the physical activity processed;
- displaying the processed data representative of the physical activity on a screen (11) of the electronic equipment (1).

2. Method according to claim 1, further comprising:
a step of storing person-specific parameters in a memory space of the portable electronic equipment (1), or accessible by the portable electronic equipment (1) or by a data processing module;
said data processing using said person-specific parameters to determine said data representative of physical activity processed in a personalised fashion for the person.

3. Method according to one of claims 1 or 2, further comprising:
storing calibration parameters specific to the mechanical wristwatch in a memory space of the portable electronic equipment (1), or accessible by the portable electronic equipment (1) or by a data processing module;
said data processing using said calibration parameters specific to the mechanical wristwatch to determine said data representative of the physical activity calibrated for the mechanical wristwatch.

4. Method according to one of claims 1 to 3, said mechanical sensor (21) being a movement sensor, such as a pedometer, or an accelerometer.

5. Method according to claim 4, comprising a step of integrating the movements undergone by the movement sensor from a time of resetting to zero, and displaying by means of said analogue indicator a value corresponding to this integration.

6. Method according to one of claims 2 to 5, said data processed using the person's specific parameters indicating a distance travelled and/or a number of calories expended.

7. Method according to one of claims 1 to 6, comprising a step of automatic and periodic zero-setting of the data displayed on the dial, or a step of manual zero-setting of the data displayed on the dial (22).

8. Method according to one of claims 1 to 7, in which the data displayed is incremented with each movement of the wearer or with each movement of the wearer in a given direction or sense, and only decremented by voluntary action by the user or automatically at periodic intervals.

9. System for measuring and displaying data related to a person's physical activity, comprising:
- a wristwatch (2) fitted with a mechanical sensor (21) for capturing data representative of the person's physical activity when wearing the wristwatch, the functioning of said mechanical sensor (21) being exclusively mechanical, and an analogue indicator (20) for displaying this data on a dial of the wristwatch;
portable electronic equipment with a camera (10);
a module for analysing images (31) captured by said camera (10), allowing an analysed data corresponding to the data displayed on the dial to be determined when the image comprises a photo of the dial;
a processing module (32) for processing said analysed data determined by the image analysis module and generating processed data;
a display module (33) for displaying the processed data on a screen.

10. System according to claim 9, said mechanical sensor (21) being a movement sensor, for example a pedometer or an accelerometer.

11. System according to one of claims 9 to 10, comprising a member for automatically and periodically setting to zero the data displayed on the dial (22).

12. System according to one of claims 9 to 11, comprising a member for manually setting to zero the data displayed on the dial (22).

13. System according to one of claims 9 to 12, comprising:
a memory space for storing person-specific parameters in the portable electronic equipment (1), or accessible by the portable electronic equipment (1) or by said data processing module;
the data processing module being adapted to use said person-specific parameters to determine said data representative of physical activity processed in a personalised fashion for the person.

14. System according to one of claims 9 to 13, further comprising:
a memory space for storing calibration parameters specific to the mechanical wristwatch in the portable electronic equipment (1), or accessible by the portable electronic equipment (1) or by said data processing module;
said data processing module being configured to use said calibration parameters specific to the mechanical wristwatch to determine said data representative of the physical activity calibrated for the mechanical wristwatch.
